(19) Europäisches Patentamt
European Patent Office
Office européen des brevets

(11) Publication number: 0 249 249 B1

(12) **EUROPEAN PATENT SPECIFICATION**

(45) Date of publication of patent specification: **02.01.92**

(51) Int. Cl.5: **C07C 43/174**, C07C 43/192, C07C 43/225, C07C 69/12

(21) Application number: **87108735.9**

(22) Date of filing: **26.10.84**

(60) Publication number of the earlier application in accordance with Art.76 EPC: **0 145 207**

# (54) Glycerol derivatives.

(30) Priority: **31.10.83 US 547297**
**05.10.84 US 657211**

(43) Date of publication of application:
**16.12.87 Bulletin 87/51**

(45) Publication of the grant of the patent:
**02.01.92 Bulletin 92/01**

(84) Designated Contracting States:
**AT BE CH DE FR GB IT LI LU NL SE**

(56) References cited:

**JOURNAL OF MEDICINAL CHEMISTRY, vol. 26, no. 5, May 1983, pages 759-761, American Chemical Society; J.C. MARTIN et al.: "9-[(1,3-Dihydroxy-2-propoxy)methyl]guanine: A new potent and selective antiherpes agent"**

(73) Proprietor: **WARNER-LAMBERT COMPANY**
**201 Tabor Road**
**Morris Plains New Jersey 07950(US)**

(72) Inventor: **Sircar, Jagadish C.**
**3615 Charter Place**
**Ann Arbor Michigan 48104(US)**
Inventor: **Suto, Mark J.**
**3410 Brentwood Court**
**Ann Arbor Michigan 48104(US)**
Inventor: **Schwender, Charles F.**
**6455 Walsh Road**
**Dexter Michigan 48130(US)**

(74) Representative: **Jones, Michael Raymond et al**
**HASELTINE LAKE & CO. Hazlitt House 28 Southampton Buildings Chancery Lane**
**London WC2A 1AT(GB)**

EP 0 249 249 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compounds of the formula

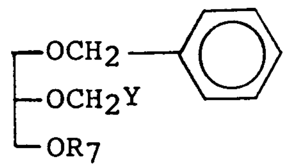

wherein Y is acetyloxy or chloro; and $R_7$ is alkyl of one to eight carbon atoms, cycloalkyl of five to seven ring members, cycloalkylalkyl, aryl or aralkyl provided that $R_7$ is not an unsubstituted benzyl radical. Preferably $R_7$ is alkyl of two to eight carbon atoms, cyclopentyl, cyclohexyl, cyclopentyl-methyl, cyclohexylmethyl, phenyl or a substituted benzyl.

This application is a third divisional of European Patent Application No. 84307373.5.

The compounds of the present invention are useful as novel intermediates in the preparation of a compound of the formula

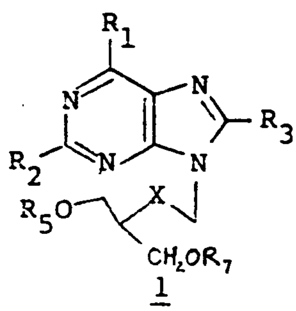

1

wherein $R_1$ is OH or SH; $R_2$ is hydrogen or NHR in which R is hydrogen or $COR_6$ where $R_6$ is alkyl of one to four carbon atoms, aryl or arylalkyl; $R_3$ is hydrogen; X is O or S; and $R_5$ is hydrogen; or a tautomeric isomer thereof, or a pharmaceutically acceptable acid or base addition salt thereof.

The compounds of formula 1 as defined above are included in the subject matter of European Patent Application No.87108734.2, which is a second divisional of European Patent Application No. 84307373.5. They are useful for treating autoimmune diseases such as arthritis, systemic lupus erythematosus, inflammatory bowel diseases, transplantation, juvenile diabetes, myasthenia gravis, multiple sclerosis as well as viral infections and cancer by administering an effective amount of the formula 1 in unit dosage form.

The term "alkyl of 1-8 carbon atoms" means a straight or branched hydrocarbon chain up to 8 carbon atoms such as, for example, methyl, ethyl, propyl, isopropyl, butyl, isobutyl, sec-butyl, tertiary-butyl, or octyl.

The term "cycloalkyl of five to seven ring members" means cyclopentyl, cyclohexyl, or cycloheptyl.

The term "cycloalkylalkyl" means a cyclopentyl, cyclohexyl, or cycloheptyl radical attached to an alkyl chain of up to four carbon atoms, straight or branched, such as, for example, cyclohexylmethyl or cyclohexylethyl.

The term "aryl" includes unsubstituted and substituted aromatic rings such as phenyl or phenyl substituted by halo, e.g. fluoro, chloro, bromo or alkyl of 1-4 carbon atoms, such as methyl or ethyl, hydroxy, alkoxy of 1-4 carbon atoms, such as methoxy or ethoxy, or trifluoromethyl.

The term "aralkyl" means an aromatic ring attached to an alkyl chain of up to 4 carbon atoms, such as unsubstituted or substituted phenylethyl or benzyl where the substituents on the aromatic ring may be the same as defined above.

The compounds of formula 1 may also be used as intermediates in the preparation of a compound of formula 5, disclosed in European Patent Application No. 84307373.5, according to the following scheme:

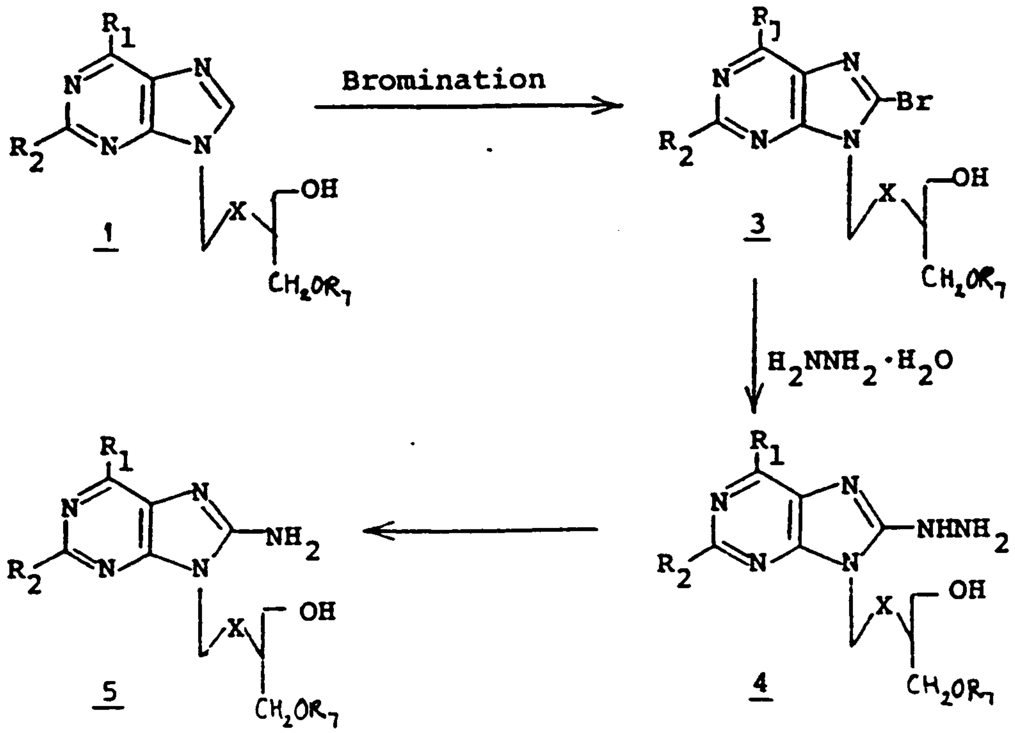


Treatment of a compound of formula 1 with N-bromosuccinimide in acetic acid, DMF or methanol produces a compound of formula 3 which, when treated with hydrazine hydrate, gives the hydrazine of formula 4 or directly the 8-amino derivative of formula 5. The reaction of the 8-bromo compound with hydrazine may or may not proceed entirely to the 8-amino compound. Thus, when the 8-hydrazine compound is obtained, it may be further reacted with Raney nickel to allow the reduction to go to completion and afford the desired 8-amino compound. Compounds of formula 5 wherein $R_1$, $R_2$, and $R_7$ have been defined according to compounds of formula 1 may be further converted by known methods to provide $R_7$ substituents of formula 1 or, for example, where $R_1$ is OH, converting said compound to a compound of formula 1 where $R_1$ is SH by known means.

The compounds of the present invention and of the formulae 1, 3, 4, and 5, shown above, may also be prepared by the following schematic sequences of reaction steps as illustrated in Schemes 1 and 2. The numbers in parentheses toward the end of each reaction scheme correspond to the compounds as defined above. A more detailed description of the reaction steps is provided in the Examples.

Scheme 1

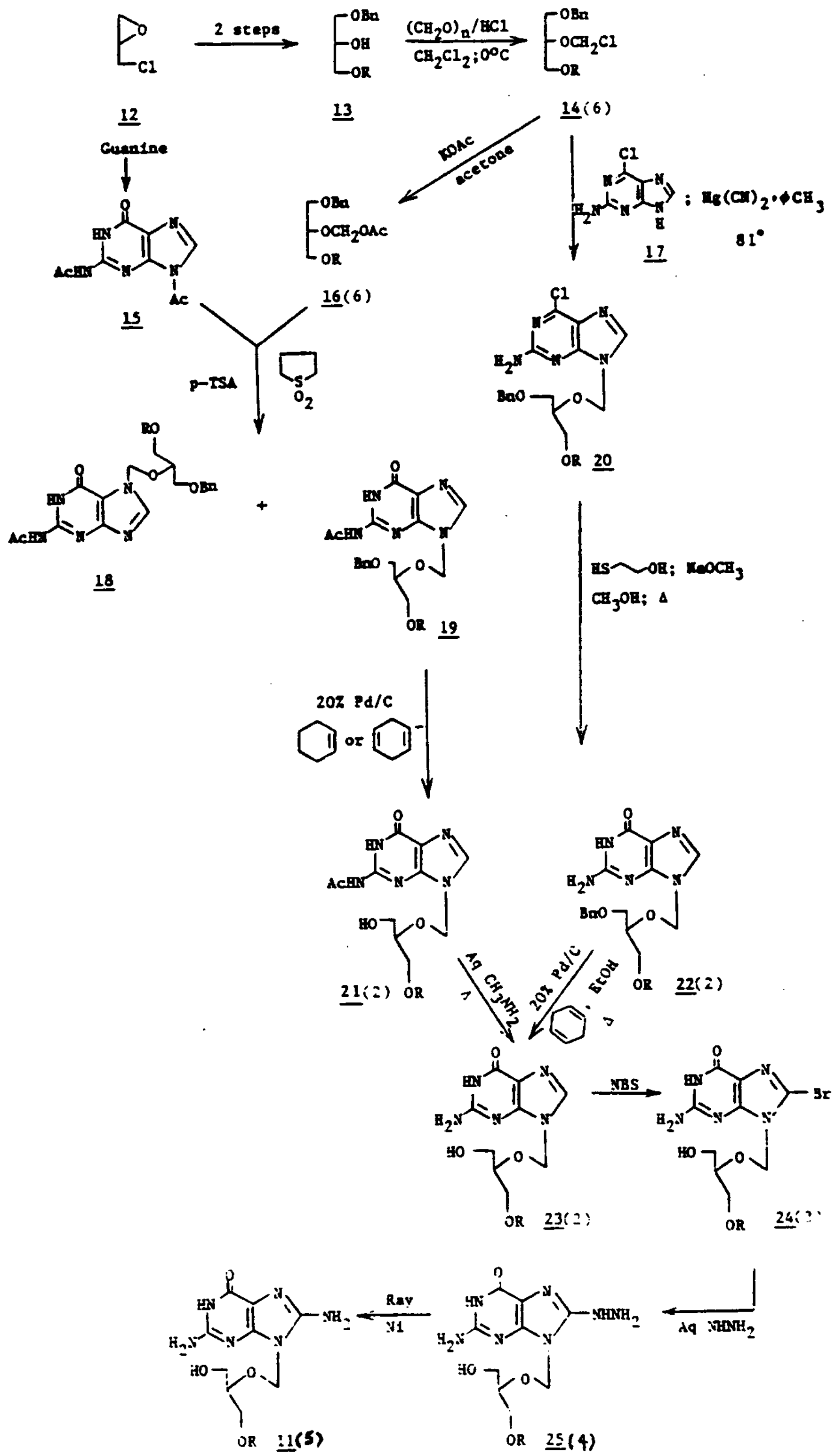
2 steps
$(CH_2O)_n/HCl$
$CH_2Cl_2$;0°C
12
13
14(6)
Guanine
KOAc
acetone
; $Hg(CN)_2 \cdot \phi CH_3$
81°
17
15
16(6)
p-TSA
20
18
19
HS⁀OH; NaOCH₃
$CH_3OH$; Δ
20% Pd/C
or
21(2)
22(2)
Aq $CH_3NH_2$
Δ
20% Pd/C
, EtOH
Δ
NBS
23(2)
24(2)
Ray
Ni
Aq $NHNH_2$
11(5)
25(4)

Scheme 2

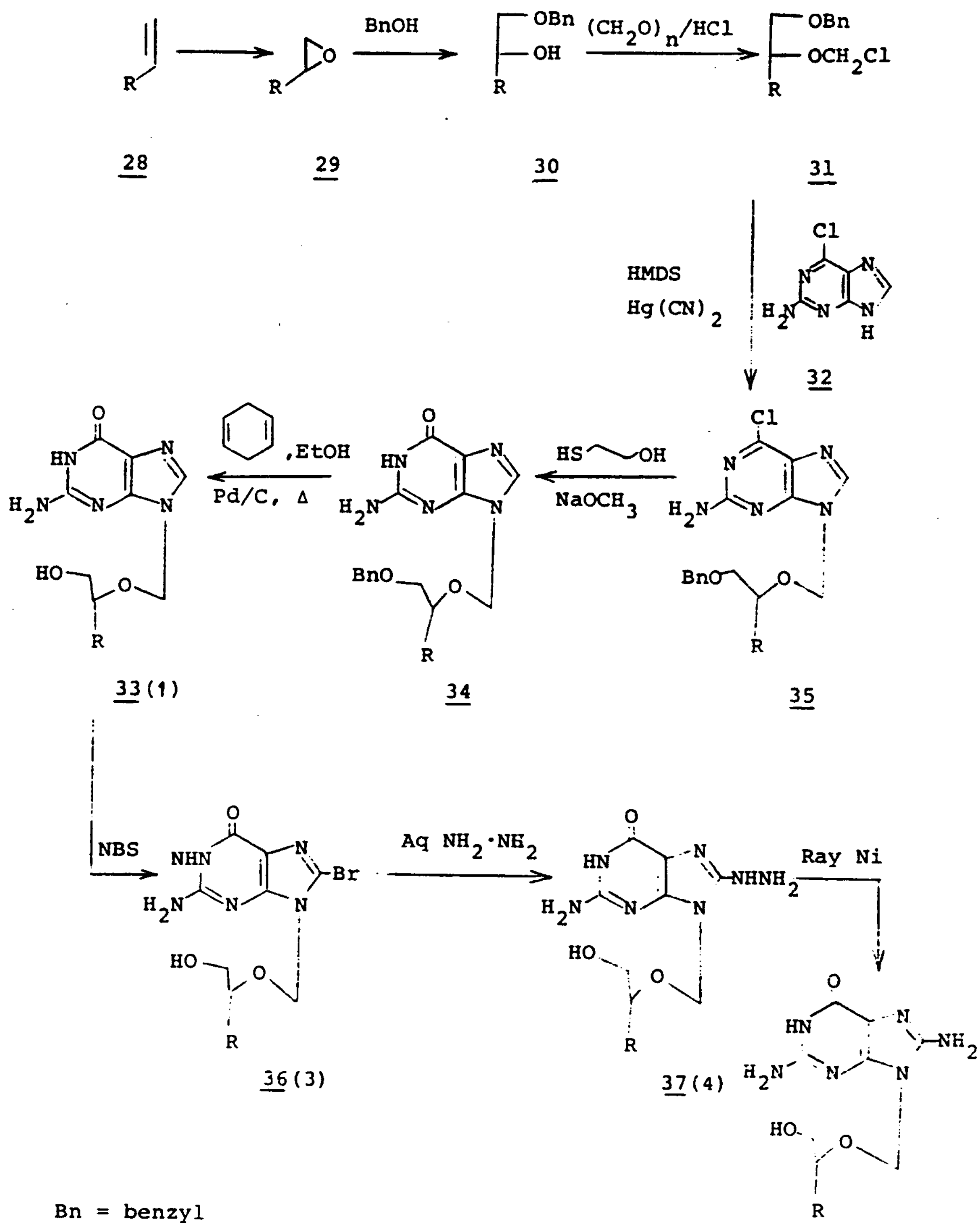


28 29 30 31 32 35 34 33(1) 36(3) 37(4) 26(5)

Bn = benzyl

The present invention is further illustrated by way of some of the following examples. Some of the following examples are directed to compounds claimed in the second divisional, European Patent Application No. 87108734.2, i.e. end products in the production of which the compounds of the present invention are intermediates. The remaining examples are directed to compounds claimed in European Patent Application No. 84307373.5 and a first divisional application thereof, European Patent Application No.

87108733.4. However, in view of the technical interrelationship between the subject matter claimed in all four applications, the inclusion of all of the abovementioned examples in the present specification assists in a clearer understanding of the methods and concepts involved in the production of the compounds according to the present invention, and of their use in the production of end products.

EXAMPLE 1

2-Amino-8-bromo-9-[(2-hydroxyethoxy)methyl]-9H-purin-6-ol[a]

N-bromosuccinimide (0.415 g; 2.3 mmol) is added to a solution of acycloguanosine (0.5 g; 2.2 mmole) (prepared according to British Patent 1,567,671) in acetic acid (7 ml) and the mixture stirred at room temperature for 20 hours. The solution is then diluted with water (20 ml) and the precipitated product is filtered, washed, and triturated with hot water to give 0.25 g of white solid, mp > 300 °C.

EXAMPLE 1A

The procedure described in Example 1 is repeated to prepare the following 8-bromo-9-substituted guanines starting from appropriate 9-substituted guanines in each case using acetic acid, methanol or DMF as solvent:

2-amino-8-bromo-9-[[2-(heptyloxy)-1-(hydroxymethyl)ethoxy]methyl]-1,9-dihydro-6H-purin-6-one, mp >250 °C, dec;

2-amino-8-bromo-9-[[2-(hexyloxy)-1-(hydroxymethyl)ethoxy]methyl]-1,9-dihydro-6H-purin-6-one, mp >250 °C, dec;

2-amino-8-bromo-9-[[2-butoxy-1-(hydroxymethyl)ethoxy]methyl]-9H-purin-6-ol, mp >200 °C;

2-amino-8-bromo-1,9-dihydro-9-[[1-(hydroxymethyl)-2-(octyloxy)ethoxy]methyl]-6H-purin-6-one, mp 223-226 °C, dec;

2-amino-8-bromo-9-[[2-)hexyloxy)-1-(hydroxymethyl)ethoxy]methyl]-1,9-dihydro-6H-purin-6-one, mp 212-214 °C;

2-amino-8-bromo-9[[2-ethoxy-1-(hydroxymethyl)ethoxy]methyl]-1,9-dihydro-6H-purin-6-one, mp 217-219 °C, dec;

2-amino-8-bromo-1,9-dihydro-9-[[1-(hydroxymethyl)-2-(pentyloxy)ethoxy]methyl]-6H-purin-6-one, mp >250 °C, dec;

2-amino-8-bromo-9-[[2-(cyclohexylmethoxy)-1-(hydroxymethyl)ethoxy]methyl]-1,9-dihydro-6H-purin-6-one, mp 210-212 °C (dec);

2-amino-8-bromo-1,9-dihydro-9-[[1-(hydroxymethyl)-2-(phenoxy)ethoxy]methyl]-6H-purin-6-one, mp 218-219 °C, dec;

2-amino-8-bromo-1,9-dihydro-9-[[2-hydroxy)-1-[(4-methoxyphenoxy)methyl]ethoxy]methyl]-6H-purin-6-one; mp 205-210 °C, dec;

2-amino-8-bromo-1,9-dihydro-9-[[1-(hydroxymethyl)-2-(4-methylphenoxy)ethoxy]methyl]-6H-purin-6-one, mp 207-208 °C, dec;

2-amino-8-bromo-1,9-dihydro-9-[[2-(4-chlorophenoxy)-1-(hydroxymethyl)ethoxy]methyl]-6H-purin-6-one, and

2-amino-8-bromo-1,9-dihydro-9-[[[1-(hydroxymethyl)nonyl[oxy]methyl]-6H-purin-6-one, mp 211-212 °C, dec.

EXAMPLE 2

2,8-Diamino-9-[(2-hydroxyethoxy)methyl)]-9H-purin-6-ol

The crude 2-amino-8-bromo-9-[(2-hydroxyethoxy)methyl]-9H-purin-6-ol from acycloguanosine (3.17 g; 0.14 mol) is suspended in water (10 ml) and 97% hydrazine (4 ml) is added to the mixture. The mixture is refluxed for 48 hours, cooled and filtered to give a white solid (1.6 g) which is triturated with hot water (75 ml) to give the analytical sample (1.5 g), mp > 300 ° dec.

EXAMPLE 3

2-[(2-Amino-8-bromo-6-hydroxy-9H-purin-9-yl)methoxy]-1,3-propanediol

a.... The structure of this compound is disclosed in Biochem. Pharm., 30, 3071-3077 (1981) by P.M. Keller, et. al..

N-bromosuccinimide (0.375 g; 2.1 mmol) is added to a solution of 9′-[(1,3-dihydroxy-2-propoxy)methyl]-guanine (0.5 g; 1.9 mmole) [prepared according to J. C. Martin; C. A. Dvorak, D. F. Smee, T. R. Matthews, and J. P. H. Verheyden, J Med Chem 26, 759-761 (1983)] in acetic acid (7 ml). The suspension is stirred for 1.5 hours at room temperature and then diluted with water (60 ml). The aqueous solution is concentrated and the residue is recrystallized from water to give 0.44 g of the product; mp > 300° dec.

EXAMPLE 4

2-[(2,8-Diamino-6-hydroxy-9H-purin-9-yl)methoxy]-1,3-propanediol

A mixture of 2-[(2-amino-8-bromo-6-hydroxy-9H-purin-9-yl)methoxy]-1,3-propanediol (13.7 g; 41 mmole) and 97% hydrazine (6.07 ml) in water (300 ml) is heated to reflux for 48 hours. At the end of this time, the solution is cooled and filtered to give 9.15 g of crude solid. The crude product is suspended in water (120 ml) and Raney nickel (9 g) is added. The mixture is heated at reflux for 6 hours, filtered hot and cooled. The crystals are collected and dried to give 7.15 g of the product, mp > 280° dec.

EXAMPLE 4A

The procedure described in Example 4 is repeated to prepare the following 8-amino-9-substituted guanines starting from appropriate 8-bromo-9-substituted guanines in each case using methoxyethanol as a cosolvent as necessary to make a homogeneous reaction mixture:
2,8-diamino-9-[[2-ethoxy-1-(hydroxymethyl)ethoxy]methyl]-9H-purin-6-ol, mp >220° C, dec;
2,8-diamino-9-[[2-(hexyloxy)-1-(hydroxymethyl)ethoxy]methyl]-1,9-dihydro-6H-purin-6-one, mp >265° C, dec;
2,8-diamino-9-[[2-butoxy-1-(hydroxymethyl)ethoxy]methyl]-9H-purin-6-ol, mp >240° C, dec;
2,8-diamino-1,9-dihydro-9-[[1-(hydroxymethyl)-2-(pentyloxy)ethoxy]methyl]-6H-purin-6-one, mp 274-277° C, dec;
2,8-diamino-9-[[2-(heptyloxy)-1-(hydroxymethyl)ethoxy]methyl]-1,9-dihydro-6H-purin-6-one, mp >260° C, dec;
2,8-diamino-9-[[2-(hexyloxy)-1-(hydroxymethyl)ethoxy]methyl]-1,9-dihydro-6H-purin-6-one, mp 260-265° C, dec;
2,8-diamino-9-[[2-(cyclohexylmethoxy)-1-(hydroxymethyl)ethoxy]methyl]-1,9-dihydro-6H-purin-6-one, mp 242-247° C, dec;
2,8-diamino-1,9-dihydro-9-[[1-(hydroxymethyl)-2-(octyloxy)ethoxy]methyl]-6H-purin-6-one, mp >265° C, (dec);
2,8-diamino-1,9-dihydro-9-[[1-(hydroxymethyl)-2-(phenoxy)ethoxy]methyl]-6H-purin-6-one, mp 265-271° C, dec;
2,8-diamino-1,9-dihydro-9-[[2-hydroxy-1-[(4-methoxyphenoxy)methyl]ethoxy]methyl]-6H-purin-6-one;
2,8-diamino-1,9-dihydro-9-[[1-hydroxymethyl)-2-(4-methylphenoxy)ethoxy]methyl]-6H-purin-6-one; mp > 250° C, dec;
2,8-diamino-1,9-dihydro-9-[[2-(4-chlorophenoxy)-1-hydroxymethyl)ethoxy]methyl]-6H-purin-6-one, and
2,8-diamino-1,9-dihydro-9-[[[1-(hydroxymethyl)nonyl]oxy]methyl]-6H-purin-6-one.

EXAMPLE 5

9-[[2-Benzyloxy-1-(benzyloxymethyl)-ethoxy]methyl]-8-hydrazine-guanine

A mixture of 9-[[2-benzyloxy-1-(benzyl oxymethyl)-ethoxy]methyl]guanine (2.1 g) [prepared according to K. K. Ogilvie, V. O. Cheriyan, B. K. Radatus, K. O. Smith, K. S. Galloway, and W. L. Kennell, Can J Chem, 60, 3005 (1982)] and N-bromosuccinimide (0.94 g) in acetic acid (21 ml) is stirred overnight and then is diluted with water and extracted with chloroform. The chloroform extract is dried and concentrated to give 2.3 g of yellow oil. The crude oil is suspended in ethanol (100 ml) and treated with 95% hydrazine. The solution is heated to reflux for 24 hours. The reaction mixture is then cooled and the product (0.75 g) filtered and dried, mp > 210° dec.

EXAMPLE 6

8-Amino-9-[[2-benzyloxy-1-(benzyloxymethyl)-ethoxy]methyl]-guanine

A mixture of 9-[[2-benzyloxy-1-(benzyloxymethyl)-ethoxy)methyl]-8-hydrazine-guanine (0.45 g; 0.98 mmol), water (40 ml), ethanol (40 ml), ammonium hydroxide (20 ml) and Raney nickel (1 g) is heated to reflux for 24 hours. The catalyst is filtered off and the filtrate concentrated to a solid which is recrystallized from ethanol to give 0.16 g of analytical sample, mp 255-260° dec.

EXAMPLE 7

N-[9-[[1-(Butoxymethyl)-2-(phenylmethoxy)ethoxy]methyl]-6-hydroxy-9H-purin-2-yl]acetamide

Dry HCl (g) is bubbled into a stirred mixture of paraformaldehyde (1.45 g, 0.048 mol) and 1-butoxy-3-(phenylmethoxy)-2-propanol (5.0 g, 0.021 mol) in methylene chloride (57 ml) at 0° C until all the solid is dissolved. The resulting solution is stored at 0° C for 16 hours, dried over $MgSO_4$, and then evaporated to give chloromethyl glycerol ether as a very unstable clear oil. The clear oil is then added dropwise to a stirred mixture of potassium acetate (5.0 g, 0.051 mol) in acetone (60 ml). The mixture is stirred for 16 hours at room temperature and then filtered and evaporated. The residual oil is dissolved in toluene, washed with saturated $NaHCO_3$ and water, dried, and evaporated to give the acetoxy derivative as an oil (5.6 g) which is immediately used for condensation with diacetylguanine.

A mixture of diacetylguanine (4.6 g, 0.0195 mol) and crude acetoxy derivative from above (5.6 g), p-toluene sulfonic acid (43 mg) and sulfolane (5 ml) is heated to 95° C under nitrogen atmosphere for 72 hours. At 24 hours and 48 hours, additional amounts of p-toluene sulfonic acid (20 mg each) are added. After 72 hours, the mixture is cooled, diluted with toluene and filtered. The filtrate is concentrated, chromatographed, and recrystallized from toluene to provide the desired product (1.33 g), mp 139-141° C.

EXAMPLE 8

The procedure described in Example 7 is repeated to prepare the following guanine-2-acetamide derivatives, starting from diacetylguanine and appropriate 1-(alkoxy or alkyl or substituted phenoxy)-3-(phenylmethoxy)-2-propanols in each case.

N-[6,9-dihydro-9-[[1-[(octyloxy)methyl]-2-(phenylmethoxy)ethoxy]methyl]-6-oxo-1H-purin-2-yl]acetamide, mp 127-132° C;

N-[6,9-dihydro-6-oxo-9-[[1-(phenoxymethyl)-2-(phenylmethoxy)ethoxy]methyl]-1H-purin-2-yl]acetamide, mp 144-146° C, and

N-[9-[[1-(ethoxymethyl)-2-(phenylmethoxy)ethoxy]methyl]-6,9-dihydro-6-oxo-1H-purin-2-yl]acetamide, mp 131-133° C, dec.

EXAMPLE 9

2-Amino-9-[[2-butoxy-1-(hydroxymethyl)ethoxy]methyl]-9H-purin-6-ol

A mixture of N-[9-[[1-(butoxymethyl)-2-(phenylmethoxy)ethoxy]methyl]-6-hydroxy-9H-purin-2-yl]-acetamide (1.15 g, 25.9 mmol), 20% palladium on carbon (0.2 g), cyclohexene (20 ml), and ethanol (10 ml) is heated at reflux under $N_2$. After 8 and 20 hours, additional amounts of catalyst (0.1 g) are added. After 36 hours, the solution is cooled, filtered through celite, and the filter cake is washed with DMF/ethanol. The filtrates are combined, refiltered and concentrated. The residue is mixed with aq. methyl amine (20 ml) and the mixture is heated at reflux for two hours, filtered and concentrated. The residue is recrystallized from water to give the desired product (0.7 g), mp 208-211° C.

EXAMPLE 10

The procedure described in Example 9 is repeated to prepare the following 9-substituted guanine derivatives, starting from N-[9-substituted-6-hydroxy-9H-purin-2-yl]acetamides in each case. Cyclohexene and cyclohexadiene can either be used in the transfer hydrogenation reaction:

2-amino-9-[[2-ethoxy-1-(hydroxymethyl)ethoxy]methyl]-1,9-dihydro-6H-purin-6-one, mp 206-209° C;

2-amino-1,9-dihydro-9[[1-(hydroxymethyl)-2-phenoxyethoxy]methyl]-6H-purin-6-one, mp 195-198° C, and

2-amino-1,9-dihydro-9-[[1-(hydroxymethyl)-2-(octyloxy)ethoxy]methyl]-6H-purin-6-one, mp 227-230° C.

EXAMPLE 11

2-Amino-9-[[1-[(heptyloxy)methyl]-2-[phenylmethoxy)ethoxy]methyl]-1,9-dihydro-6H-purin-6-one

A mixture of 2-amino-6-chloropurine (Aldrich Chemical Co.) 11.2 g, 0.066 mol) hexamethyldisilazane (160 ml), and ammonium sulfate (1.09 g) is refluxed for 2.5 hours and then cooled, concentrated and pumped to dryness. The residue is dissolved in dry toluene (210 ml) and is treated with $Hg(CN)_2$. The mixture is heated to 80°C and a solution of 2-(chloromethoxy)-1-(heptyloxy-3-(phenylmethoxy) propane (prepared from 1-(heptyloxy)-3-(phenylmethoxy)-2-propanol (19 g, 0.068 mol), paraformaldehyde (4 g) and dry HCl (g) in $CH_2Cl_2$ (160 ml) as described in the first part of Example 7, in toluene (210 ml) is added to the solution and heated to 80-85°C for 2.5 hours. The mixture is cooled, concentrated, and diluted with $CH_2Cl_2$ (1.0 L) and is allowed to stand overnight. The $CH_2Cl_2$ solution is filtered, washed with 30% KI, 10% potassium carbonate solution and water. The organic layer is dried and concentrated. The residue is chromatographed over silica gel column using a high pressure liquid chromatographic instrument (Waters Prep 500). The column is eluted with ethyl acetate and hexane (1:1) to give the condensation product (i.e., chloropurine derivative) (6.45 g) which is hydrolysed as follows.

A mixture of the above chloropurine derivative (6.42 g, 0.0139 mol) methanol (150 ml) and sodium methoxide (3 g, 0.056 mol) is treated with mercaptoethanol (4.4 ml) and water (0.26 ml). The mixture is then heated to reflux under nitrogen for two hours and then an additional amount of sodium methoxide (1.9 g) is added. The reaction mixture is heated to reflux for an additional 4.0 hours, cooled, and concentrated to about 50 ml. The concentrate is diluted with water (120 ml) and the solution is acidified to pH 6.0. The solid precipitate is filtered, washed with water, and dried. The crude product is then recrystallized from methanol/water to give an analytical sample (4.25 g), mp 185-187°C.

EXAMPLE 12

The procedure described in Example 11 is repeated to prepare the following 9-substituted guanines starting from 2-amino-6-chloropurine and appropriate 1-(alkoxy or substituted phenoxy or alkyl)-3-(phenylmethoxy)-2-propanols in each case:

2-amino-9-[1-[(cylcohexylmethoxy)methyl]-2-(phenylmethoxy)ethoxy]methyl]-1,9-dihydro-6H-purin-6-one, mp 198-201°C;

2-amino-9-[1-[(hexyloxy)methyl]-2-(phenylmethoxy)ethoxy]methyl]-1,9-dihydro-6H-purin-6-one; mp 192-194°C.

2-amino-1,9-dihydro-9-[1-[(pentyloxy)methyl]-2-(phenylmethoxy)ethoxy]methyl]-6H-purin-6-one, mp 192-194°C;

2-amino-1,9-dihydro-9-[-1-[(octyloxy)methyl]-2-(phenylmethoxy)ethoxy]methyl]-6H-purin-6-one, mp 184-186°C;

2-amino-1,9-dihydro-9-[1-(phenoxy)methyl)-2-(phenylmethoxy)ethoxy]methyl]-6H-purin-6-one;

2-amino-1,9-dihydro-9-[[[1-[(phenylmethoxy)methyl]hexyl]oxy]methyl-6H-purin-6-one, mp 206-208°C;

2-amino-1,9-dihydro-9[[[1-[(phenylmethoxy)methyl]nonyl]oxy]methyl-6H-purin-6-one, mp 205-207°C;

2-amino-9-[1-[(4-chlorophenoxy)methyl]-2-[phenylmethoxy]ethoxy]methyl]-1,9-dihydro-6H-purin-6one, mp >210°C;

2-amino-1,9-dihydro-9-[1-[(4-methoxyphenoxy)methyl]-2-[phenylmethoxy]ethoxy]methyl]-6H-purin-6-one, mp 150-156°C, and

2-amino-1,9-dihydro-9-[1-[(4-methylphenoxy)methyl]-2-[phenylmethoxy]ethoxy]methyl]-6H-purin-6-one, mp 198-200°C.

EXAMPLE 13

2-Amino-9-[[2-(heptyloxy)-1-(hydroxymethyl)ethoxy]methyl]-1,9-dihydro-6H-purin-6-one

A mixture of 2-amino-9-[1-[(heptyloxy)methyl]-2-(phenylmethoxy)ethoxy]methyl]-1,9-dihydro-6H-purin-6-one (3.9 g, 8.97 mmol), ethanol (200 ml), cyclohexadiene (87 ml, 92.3 mmol), and 20% palladium on charcoal (1.5 g) is heated to reflux under nitrogen atmosphere. After seven hours an additional amount of 20% palladium on charcoal (0.5 g) is added and the mixture is heated to reflux for a total of 18 hours. The mixture is filtered hot and then allowed to cool. The solid formed is collected and dried to give the desired purine (1.95 g), mp 224-225°C.

EXAMPLE 14

The procedure described in Example 13 is repeated to prepare the following 9-substituted guanines starting from appropriate phenylmethoxy derivatives described in Example 11 and 12.
2-amino-1,9-dihydro-9-[[[1-(hydroxymethyl)hexyl]oxy]methyl]-6H-purin-6-one, mp 228-229 ° C;
2-amino-1,9-dihydro-9-[[[1-(hydroxymethyl)nonyl]oxy]methyl]-6H-purin-6-one, mp >250 ° C, dec;
2-amino-9-[[2-(ethoxy)-1-(hydroxymethyl)ethoxy]methyl]-1,9-dihydro-6H-purin-6-one, mp 206-209 ° C;
2-amino-9-[[2-(butoxy)-1-(hydroxymethyl)ethoxy]methyl]-9H-purin-6-ol, mp 208-211 ° C;
2-amino-9-[[2-(hexyloxy)-1-(hydroxymethyl)ethoxy]methyl]-1,9-dihydro-6H-purin-6-one;
2-amino-1,9-dihydro-9-[[1-(hydroxymethyl)-2-(penyloxy)ethoxy]methyl]-6H-purin-6-one, mp 218-220 ° C;
2-amino-1,9-dihydro-9-[[1-(hydroxymethyl)-2-(octyloxy)ethoxy]methyl]-6H-purin-6-one, mp 227-230 ° C;
2-amino-9-[[2-(cyclohexylmethoxy)-1-(hydroxymethyl)ethoxy]methyl]-1,9-dihydro-6H-purine-6-one, mp > 260 ° C, dec;
2-amino-1,9-dihydro-9-[[1-(hydroxymethyl)-2-phenoxyethoxy]methyl]-6H-purin-6-one, mp 195-198 ° C;
2-amino-1,9-dihydro-9-[[1-(hydroxymethyl)-2-(4-methylphenoxy)ethoxy]methyl]-6H-purin-6one, mp 206-208 ° C;
2-amino-1,9-dihydro-9-[[2-(hydroxy-1-[(4-methoxyphenoxy)methyl]ethoxy]methyl]-6H-purin-6-one, mp 210-217 ° C, dec, and
2-amino-9-[[2-(4-chlorophenoxy)-1-(hydroxymethyl) ethoxy]methyl]-1,9-dihydro-6H-purin-6-one,

SYNTHESIS OF STARTING MATERIALS

EXAMPLE A

1-Butoxy-3-(phenylmethoxy)-2-propanol

n-Butanol (2.5 ml, 27 mmol) is added to a suspension of sodium hydride (50% in mineral oil; 1.3 g, 27 mmol) in DMF (5 ml) and the mixture is then heated to 80 ° C for 1.0 hours when all the sodium hydride is consumed. A solution of 2,3-epoxypropyl benzyl ether (benzyl glycidylether * ) (4.52 g, 27 mmol) in DMF (5 ml) is added slowly to the n-butoxide solution. The mixture is then heated to 80 ° C for 16 hours, diluted with water and extracted with ether. The ether layer is dried and concentrated to give an oil which is distilled to provide the desired product (3.1 g), bp 125-130 ° /0.8-0.5 mm.

EXAMPLE B

The procedure described in Example A is repeated to prepare the following 1-alkoxy or aryloxy-3-(phenylmethoxy)-2-propanols, starting from appropriate alkanols or phenols in each case. 1-(ethoxy)-3-(phenylmethoxy)-2-propanol, bp 92-99 ° C/ 0.25-0.3 mm;
1-(pentyloxy)-3-(phenylmethoxy)-2-propanol, bp 115-118 ° C/0.3 mm;
1-(hexyloxy)-3-(phenylmethoxy)-2-propanol, bp 123-125 ° C/0.12 mm;
1-(heptyloxy)-3-(phenylmethoxy)-2-propanol, bp 141 ° C/0.36 mm, and
1-(octyloxy)-3-(phenylmethoxy)-2-propanol, bp 150-155 ° C/0.7 mm.

EXAMPLE C

1-(Phenylmethoxy)-2-decanol

Benzyl alcohol (108 g, 1.0 mol) is added to a suspension of 50% sodium hydride-mineral oil (48 g, 1.0 mol) in DMF (200 ml) at room temperature. The mixture is then heated to 80 ° C for two hours. A solution of 1,2-epoxydecane * (85 ml) in DMF (50 ml) is added slowly to the sodium salt over 30 minutes and the mixture is then heated at 80 ° C for 20 hours. The reaction mixture is cooled, diluted with water, neutralized with acetic acid, and extracted with ether. The ether extract is concentrated to give an oil which is distilled to give the desired product (131 g), bp 178-180 ° C/4 mm.

*Benzyl glycidyl ether is prepared according to the published procedure (J. R Bacon and M. J. Collis, Chem. and Ind., 1971, 930) or purchased from commercial source.

*Purchased from Aldrich Chemical Co. Other epoxides are either purchased or prepared from olefin or epichlorohydrine as the case may be.

EXAMPLE D

The procedure described in Example C is repeated to prepare the following 1-(phenylmethoxy)-alkanols, starting from appropriate 1,2-epoxides in each case. 1-(cyclohexylmethoxy)-3-(phenylmethoxy)-2-propanol, bp 136-139°C/0.24-0.22 mm;
1-(phenylmethoxy)-2-heptanol, bp 125-130°C/3-5 mm;
1-(phenoxy)-3-(phenylmethoxy)-2-propanol, bp 148-157°C/0.32 mm;
1-(4-methylphenoxy)-3-(phenylmethoxy)-2-propanol, bp 194°C/2 mm;
1-(4-methoxyphenoxy)-3-(phenylmethoxy)-2-propanol, bp 175-184°C/0.4 mm, and
1-(4-chlorophenoxy)-3-(phenylmethoxy)-2-propanol, bp 188-190°C/1.2-1.3 mm.

**Claims**

**Claims for the following Contracting States : BE, CH, DE, FR, GB, IT, LI, LU, NL, SE**

**1.** A compound having the following general formula:

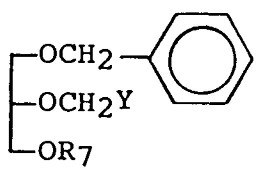

wherein: Y is an acetyloxy or a chloro radical;

$R_7$ is a $C_{1-8}$ alkyl radical, a cycloalkyl radical of from five to seven ring members, a cycloalkylalkyl, an aryl or an aralkyl radical;

the term "aryl" relates to aromatic rings which are unsubstituted or substituted by halo, alkyl of from 1 to 4 carbon atoms or alkoxy of from 1 to 4 carbon atoms;

the term "aralkyl" relates to an aromatic ring attached to an alkyl group of from 1 to 4 carbon atoms; and

the term "cycloalkylalkyl" relates to a cycloalkyl group of from five to seven ring members attached to an alkyl of from 1 to 4 carbon atoms;

provided that $R_7$ is not an unsubstituted benzyl radical.

**2.** A compound according to Claim 1, wherein: $R_7$ is a $C_{2-8}$ alkyl radical, a cyclopentyl, a cyclohexyl, a cyclopentylmethyl, a cyclohexylmethyl, a phenyl or a substituted benzyl radical.

**Claims for the following Contracting State : AT**

**1.** A process for preparing a compound having the following general formula:

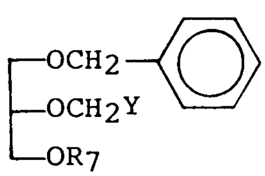

wherein: Y is an acetyloxy or a chloro radical;

$R_7$ is a $C_{1-8}$ alkyl radical, a cycloalkyl radical of from five to seven ring members, a cycloalkylalkyl, an aryl or an aralkyl radical;

the term "aryl" relates to aromatic rings which are unsubstituted or substituted by halo, alkyl of from 1 to 4 carbon atoms or alkoxy of from 1 to 4 carbon atoms;

the term "aralkyl" relates to an aromatic ring attached to an alkyl group of from 1 to 4 carbon atoms; and

the term "cycloalkylalkyl" relates to a cycloalkyl group of from five to seven ring members attached to an alkyl of from 1 to 4 carbon atoms;

provided that $R_7$ is not an unsubstituted benzyl radical;

which process comprises treating a compound having the formula

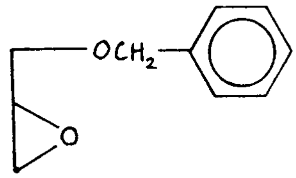

with an oxide of the formula $R_7O^-$ to give a compound having the formula

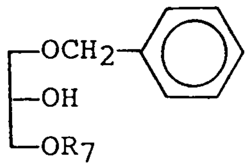

subsequently converting the hydroxyl group to a $-OCH_2Cl$ group and optionally converting the thus formed $-OCH_2Cl$ group to an acetyloxy radical.

**2.** A process according to Claim 1, wherein $R_7$ is a $C_{1-8}$ alkyl radical, a cyclopentyl, a cyclohexyl, a cyclopentylmethyl, a cyclohexylmethyl, a phenyl radical or a substituted benzyl radical.

**Revendications**
**Revendications pour les Etats contractants suivants : BE, CH, DE, FR, GB, IT, LI, LU, NL, SE**

**1.** Un composé présentant la formule générale suivante:

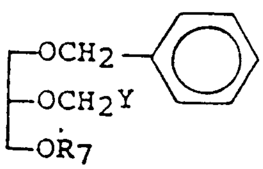

dans laquelle:

Y est un radical acétyloxy ou un atome de chlore;

$R_7$ est un radical alkyle comprenant 1 à 8 atomes de carbone, un radical cycloalkyle dont le cycle est formé de 5 à 7 atomes, un radical cycloalkylalkyle, un radical aryle ou un radical aralkyle;

- par le terme "aryle", on entend les noyaux aromatiques substitués ou non substitués par un atome d'halogène, alkyle comprenant 1 à 4 atomes de carbone ou alkoxy comprenant I à 4 atomes de carbone;
- par le terme "aralkyle", on entend un noyau aromatique lié à un radical alkyle comprenant 1 à 4 atomes de carbone; et
- par le terme "cycloalkylalkyle", on entend un radical cycloalkyle dont le cycle comprenant 5 à 7 atomes est relié à un radical alkyle comprenant 1 à 4 atomes de carbone étant entendu que $R_7$ ne peut pas être un radical benzyle non substitué.

**2.** Un composé selon la revendication 1 dans lequel $R_7$ est un radical alkyle comprenant 2 à 8 atomes de carbone, un radical cyclopentyle, un radical cyclohexyle, un radical cyclopentylméthyle, un radical cyclohexylméthyle, un radical phényle ou un radical benzyle substitué.

**Revendications pour l'Etat contractant suivant : AT**

**1.** Un procédé de préparation d'un composant présentant la formule générale suivante:

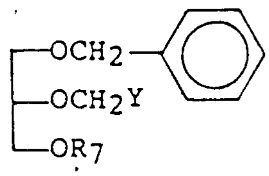

dans laquelle:

Y est un radical acétyloxy ou un atome de chlore;

$R_7$ est un radical alkyle comprenant 1 à 8 atomes de carbone, un radical cycloalkyle dont le cycle comprend 5 à 7 atomes, un radical cycloalkylalkyle, un radical aryle ou un radical aralkyle;

- par le terme "aryle", on entend les noyaux aromatiques substitués ou non substitués par un atome d'halogène ou alkyle comprenant 1 à 4 atomes de carbone ou alkoxy comprenant 1 à 4 atomes de carbone;
- par le terme "aralkyle", on entend un noyau aromatique lié à un radical alkyle comprenant 1 à 4 atomes de carbone; et
- par le terme "cycloalkylalkyle" on entend un radical cycloalkyle dont le cycle comprenant 5 à 7 atomes est relié à un radical alkyle comprenant 1 à 4 atomes de carbone étant entendu que $R_7$ ne peut pas être un radical benzyle non substitué;

lequel procédé consiste à traiter un composé présentant la formule:

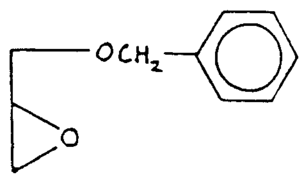

par un oxyde de formula $R_7O^-$ pour produire un composé présentant la formule:

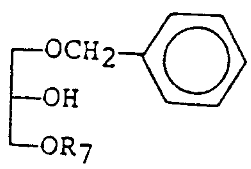

et à convertir ultérieurement le radical hydroxyle en un groupe $-OCH_2Cl$ et éventuellement à convertir le groupe $-OCH_2Cl$ ainsi formé en un radical acétyloxy.

**2.** Un procédé selon la revendication 1 dans lequel $R_7$ est un radical alkyle comprenant 2 à 8 atomes de carbone, un radical cyclopentyle, un radical cyclohexyle, un radical cyclopentylméthyle, un radical cyclohexylméthyle, un radical phényle ou un radical benzyle substitué.

**Patentansprüche**

**Patentansprüche für folgende Vertragsstaaten : BE, CH, DE, FR, GB, IT, LI, LU, NL, SE**

**1.** Verbindung der folgenden allgemeinen Formel:

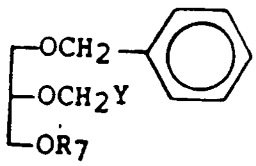

worin:
Y ein Acetyloxy oder ein Chlorrest ist;
$R_7$ ein $C_{1-8}$-Alkylrest, ein Cycloalkylrest mit fünf bis sieben Ringgliedern, ein Cycloalkylalkyl, ein Aryl oder ein Aralkylrest ist;
der Ausdruck "Aryl" aromatische Ringe, welche unsubstituiert oder substituiert durch Halogen, Alkyl mit 1 bis 4 Kohlenstoffatomen oder Alkoxy mit 1 bis 4 Kohlenstoffatomen sind, betrifft;
der Ausdruck "Aralkyl" einen aromatischen Ring, welcher an eine Alkylgruppe mit 1 bis 4 Kohlenstoffatomen gebunden ist, betrifft
und
der Ausdruck "Cycloalkylalkyl" eine Cycloalkylgruppe mit fünf bis sieben Ringgliedern, welche an ein Alkyl mit 1 bis 4 Kohlenstoffatomen gebunden ist, betrifft;
mit der Maßgabe, daß $R_7$ nicht ein unsubstituierter Benzylrest ist.

**2.** Verbindung nach Anspruch 1, worin:
$R_7$ ein $C_{2-8}$-Alkylrest, ein Cyclopentyl, ein Cyclohexyl, ein Cyclopentylmethyl, ein Cyclohexylmethyl, ein Phenyl oder ein substituierter Benzylrest ist.

**Patentansprüche für folgenden Vertragsstaat : AT**

**1.** Verfahren zur Herstellung einer Verbindung der folgenden allgemeinen Formel:

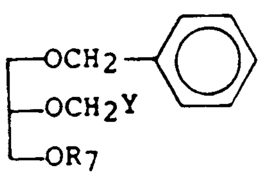

worin:
Y ein Acetyloxy oder ein Chlorrest ist;
$R_7$ ein $C_{1-8}$-Alkylrest, ein Cycloalkylrest mit fünf bis sieben Ringgliedern, ein Cycloalkylalkyl, ein Aryl oder ein Aralkylrest ist;
der Ausdruck "Aryl" aromatische Ringe, welche unsubstituiert oder substituiert durch Halogen, Alkyl mit 1 bis 4 Kohlenstoffatomen oder Alkoxy mit 1 bis 4 Kohlenstoffatomen sind, betrifft;
der Ausdruck "Aralkyl" einen aromatischen Ring, welcher an eine Alkylgruppe mit 1 bis 4 Kohlenstoffatomen gebunden ist, betrifft
und
der Ausdruck "Cycloalkylalkyl" eine Cycloalkylgruppe mit fünf bis sieben Ringgliedern, welche an ein Alkyl mit 1 bis 4 Kohlenstoffatomen gebunden ist, betrifft;
mit der Maßgabe, daß $R_7$ nicht ein unsubstituierter Benzylrest ist;
welches Verfahren die Behandlung einer Verbindung der Formel

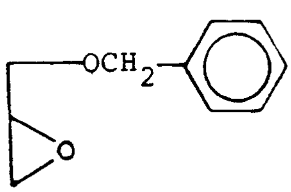

mit einem Oxid der Formel $R_7O$- zum Erhalt einer Verbindung der Formel

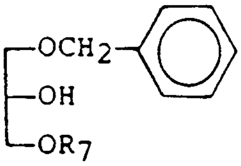

darauffolgendes Überführen der Hydroxylgruppe in eine $-OCH_2Cl$-Gruppe und gegebenenfalls Überführen der auf diese Weise gebildeten $-OCH_2Cl$-Gruppe in einen Acetyloxyrest umfaßt.

**2.** Verfahren nach Anspruch 1, worin $R_7$ ein $C_{1-8}$-Alkyrest, ein Cyclopentyl, ein Cyclohexyl, ein Cyclopentylmethyl, ein Cyclohexylmethyl, ein Phenylrest oder ein substituierter Benzylrest ist.